# EUROPEAN PATENT SPECIFICATION

(11) **EP 0 591 680 B1**
(45) Date of publication and mention of the grant of the patent: **20.05.1998**
(21) Application number: 93114104.8
(22) Date of filing: 02.09.1993
(51) Int. Cl.: A61N 1/05

(54) **Electrode system for pacemakers**
Elektrodensystem für Herzschrittmachern
Système d'électrodes pour stimulateur cardiaque

(30) Priority: 28.09.1992 SE 9202792
(43) Date of publication of application: 13.04.1994
(73) Proprietor: Pacesetter AB, 171 95 Solna (SE)
(72) Inventor: Högnelid, Kurt, S-137 38 Västerhaninge (SE); Öhman, Alf, S-162 45 Vällingby (SE)
(74) Representative: Lettström, Richard Wilhelm

(56) References cited:
- US-A- 3 737 579
- US-A- 4 624 266
- US-A- 4 628 943
- US-A- 5 143 090

## Description

This invention relates to an endocardial electrode system for pacemakers, comprising at least one endocardial ventricular electrode intended for placement in contact with heart tissue in the ventricle, in order to sense electrical activity and stimulate, and connected to a conductor extending from a distal end portion of a cable for transmitting signals to and from the electrode.

Stimulation in both single chamber and dual chamber pacemakers with ventricular stimulation, i.e. VVI and DDD pacemakers, is normally in the apex. A conventional pacemaker of this type for treating patients suffering from e.g. bradycardia requires two additional electrodes, each with its own connecting conductor, one of these electrodes being placed in the heart's atrium and the other in the ventricle. The task of the electrodes is to sense electrical activity in the heart and to emit stimulation pulses when spontaneous electrical activity ceases.

One example of an electrode system of this type is described in e.g. US-A-4.567.901. Here, the electrode cable is subdivided in a precurved area into an atrial section and a ventricular section, said sections in turn being curved so the atrial electrode and the ventricular electrode carried by them end up at the desired sites in the heart when the electrode system is implanted. Such an electrode system with a precurved electrode cable subdivided into two separate sections is a complex system which is difficult to implant in the patient.

So there has been a wish to achieve an electrode system for DDD pacemakers with the electrode conductors bundled in a single cable.

In attempts to achieve such a simplification of the electrode system, electrode systems have been used previously with the conductors bundled in a single cable, at one end of which an ordinary stimulation and sensing electrode is arranged for implantation in the conventional manner in the ventricle near the apex. This type of electrode system also comprises one or a plurality of electrodes arranged on the cable in the atrium of the heart. However, these atrial electrodes are "floating", i.e. they are normally not in direct contact with electrically active tissue, so effective stimulation is impossible, and sensing with the aid of these electrodes is more difficult.

In one attempt to bring the atrial electrode(s) into contact with the heart tissue in the atrium in order to stimulate there, the part of the cable carrying the atrial electrode(s) has been preshaped so the electrode(s) press(es) against the heart tissue in the atrium, cf US-A-4.154.247. The cable is thus preshaped into a curve, loop or the like so a ring electrode on the cable makes contact with the atrial wall. However, this preshaping of the cable makes implantation more difficult, and the contact with the atrial wall is unreliable.

US-5.143.090 discloses a cardiac lead or pacemaker cable having an elongated electrical conductor connected to an electrode head at a distal end of the conductor. An epicardial electrode mounted on the head comprises a helical wire electrode adapted to be turned into heart tissue. However, this helical wire electrode protrodes from the electrode head in a direction being perpendicular to the direction of the distal conductor end.

US-4.628.943 discloses an endocardial screw-in pacing lead comprising an insulative tubular sheath having a distal end and a proximal end. First and second conductive members, being insulated from each other, are received in and extend the length of the tubular sheath. At the distal end of this sheath the pacing lead includes a distal electrode assembly comprising a corkscrew attachment device being electrically and mechanically connected to the second conductive member, the distal end of which is electrically and mechanically connected to the proximal end of a cylindrical mounting member made of electrically conductive material and being rotatably mounted within the distal electrode assembly. A proximal end of the corkscrew attachment device is mounted to the distal end of the mounting member in an electrically conductive manner and is rotatable when the mounting member is caused to rotate by rotation of the second conductive member. When the corkscrew attachment device is rotated in a screw like manner in a forward direction through a helical spiral passageway extending through a fixed plug made of insulative material, a distal forward wire electrode section of the cork screw attachment device is screwed out of the insulating fixed plug and into heart tissue. However, no part of the helical corkscrew wire electrode is per se provided with any insulative layer, and the wire electrode is merely screwable out through the helical spiral passageway extending through the insulating fixed plug.

Studies have shown that it would be advantageous with pacemakers providing ventricular stimulation to stimulate high up in the ventricle, e.g. high up in the septum or occasionally, for practical reasons, in the superior part of the outer ventricular wall. Stimulation in the apex has proved to be capable of rapidly resolving acute problems, but some excess mortality has been observed in patients receiving this type of stimulation compared to stimulation in the upper atrial wall. Stimulation high up in the septum has been found to be similar to natural stimulation, since depolarization then comes through the septum and subsequently spreads across the ventricle with more efficient heart beats as a result.

The object of the present invention is to eliminate the shortcomings of the above-described, previously known technic and to achieve an electrode system making possible stimulation high up in the ventricle with both single and dual chamber pacemakers providing ventricular stimulation.

This object is achieved with an endocardial electrode system of the above-described type with the features stated in claim 1. Preferred embodiments of the claimed electrode system are defined in sub-claims 2-5. With a construction according to the invention, an electrode system is achieved with which the ventricular electrode is introduced into the septum or the superior part of the outer ventricular wall, i.e. the ventricular electrode will sense and excite tissue high up in the ventricle.

According to one advantageous refinement of the electrode system according to the invention, especially devised for DDD pacemakers, the system comprises an atrial electrode, intended for placement in contact with heart tissue inside the atrium for sensing electrical activity and stimulation, and connected to a conductor for transmitting signals to and from the electrode, the conductors for the electrodes being arranged in one single cable. From the site of the atrial electrode, the conductor extends to the ventricular electrode, said atrial electrode being positioned in the lower part of the atrium when the ventricular electrode has been applied high up in the ventricle. In this manner, a largely "linear" electrode system is achieved which facilitates implantation, and the atrial electrode is in contact with heart tissue in the lower part of the atrium for sensing electrical activity and stimulation in the atrium, the ventricular electrode being implanted high up in the ventricle.

According to advantageous embodiments of the electrode system according to the invention, the ventricular electrode consists of the non-insulated terminal part of the otherwise insulated needle, helical or screw-shaped conductor which protrudes from the middle of the cowl forming the atrial electrode. At the implantation the needle, helical or screw-shaped conductor pierces or is screwed into the septum down to excitable heart tissue, the cowl then pressing against the inside of the lower atrial wall. The cowl can be made of carbon, platinum or titanium nitride.

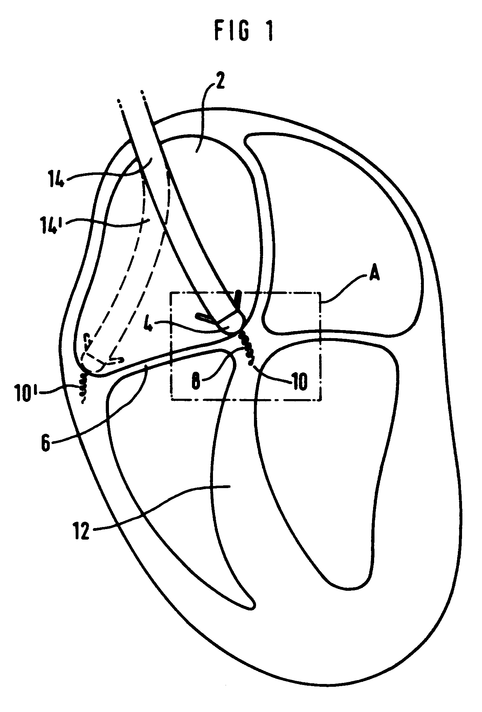

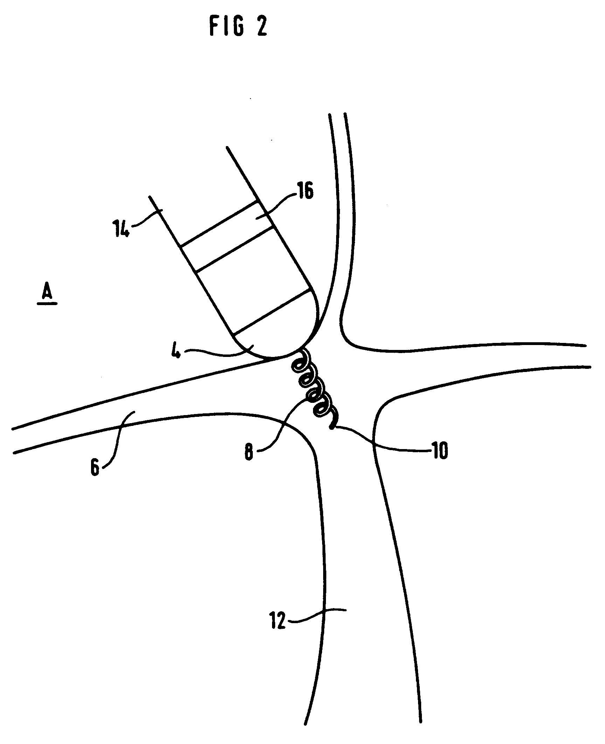

Exemplified embodiments of the electrode system according to the invention will now be described below, referring to accompanying drawings in which FIG. 1 shows a schematic cross-section of a heart with an electrode system according to the invention and FIGS. 2-3 show the central part A in FIG. 1, enlarged with two different versions of the ventricular part of the electrode system.

FIG. 1 shows a schematic cross-section of a heart with the electrode system according to the invention introduced into the right atrium 2.

The electrode system comprises an atrial electrode in the form of a cowl 4 made of carbon, platinum or titanium nitride which, in an implanted electrode system, presses against the inside of the lower part of the wall of the atrium 2, to one side of the heart valve 6. The atrial electrode can thus be used both for sensing electrical activity and stimulating the atrium.

From the middle of the cowl 4 an insulated helical or screw-shaped conductor 8 protrudes. The terminal part 10 of the helical or screw-shaped conductor 8 has no insulation and forms the ventricular electrode. The helical or screw-shaped conductor 8 is intended for screwing into the septum 12, and the helical or screw-shaped conductor 8 is long enough to permit the terminal part 10 to then reach excitable ventricular tissue. The electrode system is installed simply by rotating the electrode system around its longitudinal axis so the helical or screw-shaped conductor 8 is screwed into the septum 12.

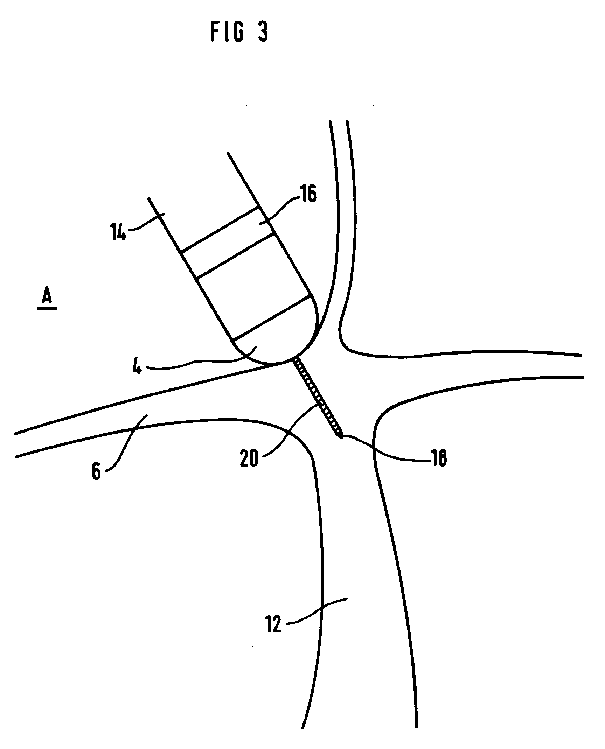

FIG. 3 shows an alternative version in which an insulated needle 20 protrudes from the middle of the cowl 4. The tip 18 of the needle 20 has no insulation and forms the ventricular electrode. When the electrode system is implanted, the needle 20 is stuck into the septum 12 so the tip 18 reaches excitable tissue.

The electrodes 4,10 and 18 thus are electrically insulated from one another, and the ventricular electrode 10, 18 senses electrical activity and stimulates tissue high up in the ventricle. As noted above, studies have shown that stimulation high up in the septum is more advantageous than stimulation in the apex, since the former stimulation more closely resembles conditions in natural stimulation.

Alternately, the ventricular electrode 10 can be introduced into the superior part of the outer ventricular wall. For practical reasons, this version, shown with dashed lines in FIG. 1, could be preferable in certain situations.

The wires from the electrodes 4, 10, 18 for transmitting signals to and from the electrodes are arranged in one single cable 14 which runs to the pulse generator and other electronic equipment in the pacemaker. Devising the electrode system with one single cable, even with DDD pacemakers, offers significant advantages, not the least at implantation, as noted above. The electrode system can then be easily introduced into the atrium via the vena cava.

The electrode system could possibly also comprise an atrial indifferent electrode 16 in the form a metal ring arranged on the cable 14, cf. FIGS. 2 and 3

## Claims

1. An endocardial electrode system for pacemakers, comprising at least one endocardial ventricular electrode (10,18) intended for placement in contact with heart tissue in the ventricle, in order to sense electrical activity and stimulate, and connected to a conductor extending from a distal end portion of a cable (14) for transmitting signals to and from the electrode, wherein the part (8,20) of the conductor nearest to the endocardial ventricular electrode (10,18) is an insulated part protruding in the direction of the longitudinal axis of the endocardial electrode system and distal cable end portion, and that this protruding insulated conductor part (8,20) is in the form of a helix, screw or needle for screwing into or puncturing the part of the atrial wall facing the septum (12) during implantation and then on into the septum or superior part of the outer ventricular wall.

2. An electrode system as claimed in claim 1, comprising at least one endocardial atrial electrode (4), intended for placement in contact with heart tissue inside the atrium (2) for sensing electrical activity and stimulation and connected to a conductor for transmitting signals to and from the electrode, the conductors to the electrodes (10,18,4) being arranged in one and the same single cable (14), **characterized** in that from the site of the atrial electrode (4) the conductor (8,20) extends to the endocardial ventricular electrode (10,18), the atrial electrode (4) being adapted to be positioned in the lower part of the atrium (2) when the endocardial ventricular electrode (10,18) has been applied high up in the ventricle.

3. An electrode system as claimed in claim 1 or 2, **characterized** in that the free terminal part of the needle, helical or screw-shaped conductor (8,20) has no insulation and forms the endocardial ventricular electrode (10,18).

4. An electrode system as claimed in claim 2 or 3, **characterized** in that the endocardial atrial electrode (4) is devised as a cowl from the middle of which the needle, helical or screw-shaped, insulated conductor (8,20) to the endocardial ventricular electrode (10,18) protrudes.

5. An electrode system as claimed in claim 4, **characterized** in that the cowl is made from carbon, platinum or titanium nitride.

## Patentansprüche

1. Ein endokardiales Elektrodensystem für Herzschrittmacher mit mindestens einer endokardialen ventrikulären, für die Plazierung in Kontakt mit Herzgewebe im Ventrikel vorgesehenen Elektrode (10, 18), um elektrische Aktivität abzufühlen und Zu stimulieren, und die mit einem sich von einem distalen Endbereich eines Kabels (14) erstreckenden Leiter verbunden ist, um Signale von und zu der Elektrode zu uberführen, wobei der Teil (8, 20) des Leiters am nächsten Zu der endokardialen ventrikulären Elektrode (10, 18) ein isoliertes, sich in Richtung der longitudinalen Achse des endokardialen Elektrodensystems und dem distalen Kabelendteil vorstehendes Teil ist und das dieses vorstehende isolierte Leiterteil (8, 20) die Form einer Spirale, Schraube oder Nadel zum Hineinschrauben in den Teil oder Punktieren des Teils der atriellen, dem Septum (12) zugewandten Wand während der Implantation und anschließend in das Septum oder den obersten Teil der äußeren ventrikulären Wand aufweist.

2. Ein Elektrodensystem nach Anspruch 1 mit mindestens einer endokardialen atriellen Elektrode (4), vorgesehen für die Plazierung in Kontakt mit Herzgewebe innerhalb des Atriums (2) zum Abfühlen elektrischer Aktivität und Stimulation und verbunden mit einem Leiter zur Überführung von Signalen zu und von der Elektrode, wobei die Leiter zu den Elektroden (10, 18, 4) in einem und dem selben einzigen Kabel (14) angeordnet sind, **dadurch gekennzeichnet**, daß vom Platz der atriellen Elektrode (4) der Leiter (8, 20) zu der ventrikulären Elektrode (10, 18) führt, wobei die atrielle Elektrode (4) dazu ausgebildet ist, im unteren Teil des Atriums (2) positioniert zu werden, wenn die ventrikuläre Elektrode (10, 18) hoch oben im Ventrikel angebracht worden ist.

3. Ein Elektrodensystem nach Anspruch 1 oder 2, **dadurch gekennzeichnet**, daß das freie Anschlußteil des nadel-, spiral- oder schraubenförmigen Leiters (8, 20) keine Isolation aufweist und die endokardiale ventrikuläre Elektrode (10, 18) bildet.

4. Ein Elektrodensystem nach Anspruch 2 oder 3, **dadurch gekennzeichnet**, daß die endokardiale atrielle Elektrode (4) als eine Kappe ausgebildet ist, von deren Mitte der nadel-, spiral- oder schraubenförmige Leiter (8, 20) zu der endokardialen ventrikulären Elektrode (10, 18) hervortritt.

5. Ein Elektrodensystem nach Anspruch 4, **dadurch gekennzeichnet**, daß die Kappe aus Kohlenstoff, Platin oder Titannitrid besteht.

## Revendications

1. Système d'électrode endocardiale pour stimulateur cardiaque, comprenant au moins une électrode (10, 18) ventriculaire endocardiale destinée à être placée en contact avec du tissu cardiaque dans le ventricule, afin de détecter l'activité électrique et de stimuler, et reliée à un conducteur partant d'une partie d'extrémité distale d'un câble (14) et destiné à transmettre des signaux à l'électrode et à en recevoir, la partie (8, 20) du conducteur la plus proche de l'électrode (10, 18) ventriculaire endocardiale étant une partie isolée faisant saillie dans la direction de l'axe longitudinal du système d'électrode endocardiale et de la partie d'extrémité distale du câble et cette partie (8, 20) de conducteur isolée faisant saillie est sous la forme d'une hélice, d'une vis ou d'une aiguille pour se visser dans la partie de la paroi auriculaire faisant face au septum (12) ou pour la perforer pendant l'implantation puis dans le septum ou dans la partie supérieure de la paroi ventriculaire extérieure.

2. Système d'électrode suivant la revendication 1, comprenant au moins une électrode (4) auriculaire endocardiale destinée à être placée en contact avec du tissu cardiaque à l'intérieur de l'oreillette (2) à des fins de détection de l'activité électrique et de stimulation et reliée à un conducteur destiné à transmettre des signaux à une électrode et à en recevoir, les conducteurs allant aux électrodes (10, 18, 4) étant disposés dans un seul et même câble (14) unique, caractérisé en ce qu'à partir du site de l'électrode (4) auriculaire, le conducteur (8, 20) s'étend vers l'électrode (10, 18) ventriculaire endocardiale, l'électrode (4) auriculaire étant agencée pour être placée dans la partie inférieure de l'oreillette (2) quand l'électrode (10, 18) endocardiale a été appliquée en haut du ventricule.

3. Système d'électrode suivant la revendication 1 ou 2, caractérisé en ce que la partie d'extrémité libre du conducteur (8, 20) en forme d'aiguille, d'hélice ou de vis n'a pas d'isolation et forme l'électrode (10, 18) ventriculaire endocardiale.

4. Système d'électrode suivant la revendication 2 ou 3, caractérisé en ce que l'électrode (4) auriculaire endocardiale est sous la forme d'un capuchon du milieu duquel fait saillie, vers l'électrode (10, 18) ventriculaire endocardiale, le conducteur (8, 20) isolé en forme d'aiguille, d'hélice ou de vis.

5. Système d'électrode suivant la revendication 4, caractérisé en ce que le capuchon est en carbone, en platine ou en nitrure de titane.
